# EUROPEAN PATENT SPECIFICATION

(11) **EP 2 158 908 B1**
(45) Date of publication and mention of the grant of the patent: **24.04.2013**
(21) Application number: 08777444.4
(22) Date of filing: 20.06.2008
(51) Int. Cl.: A61P 25/00

(54) **COMPOSITION FOR TRANSDERMAL ADMINISTRATION**
ZUSAMMENSETZUNG ZUR TRANSDERMALEN VERABREICHUNG
COMPOSITION DESTINÉE À ÊTRE ADMINISTRÉE PAR VOIE TRANSDERMIQUE

(30) Priority: 21.06.2007 JP 2007163692
(43) Date of publication of application: 03.03.2010
(73) Proprietor: Fujimoto Co., Ltd., Matsubara-shi Osaka 580-0011 (JP)
(72) Inventor: YONEDA, Fumio, Matsubara-shi Osaka 580-0011 (JP); OHDE, Hironori, Matsubara-shi Osaka 580-0011 (JP); WATANABE, Mayumi, Matsubara-shi Osaka 580-0011 (JP); SUGIMOTO, Mikiyo, Matsubara-shi Osaka 580-0011 (JP); KAMADA, Takahiro, Matsubara-shi Osaka 580-0011 (JP); HUKUMOTO, Mizue, Matsubara-shi Osaka 580-0011 (JP); TAKASE, Azusa, Matsubara-shi Osaka 580-0011 (JP); HOSHINO, Naoya, Matsubara-shi Osaka 580-0011 (JP)
(74) Representative: von Kreisler Selting Werner
(86) International application number: PCT/JP2008/061300
(87) International publication number: WO 2008/156160

(56) References cited:
- EP-A1- 0 957 080
- EP-A1- 1 170 004
- EP-A1- 1 844 773
- WO-A1-02/094368
- WO-A1-03/037407
- WO-A1-2006/057211
- WO-A1-2006/082888
- JP-A- 2000 136 187
- KNOLL J ET AL: "(-)1-(BENZOFURAN-2-YL)-2-PROPYLAMINOPENTA NE, not (-)BPAP 3/4 , A SELECTIVEENHANCER OF THE IMPULSE PROPAGATION MEDIATED RELEASE OF CATECHOLAMINES AND SEROTONIN IN THE BRAIN", BRITISH JOURNAL OF PHARMACOLOGY, NATURE PUBLISHING GROUP, BASINGSTOKE, HANTS; GB, vol. 128, no. 8, 1 January 1999 (1999-01-01), pages 1723-1732, XP001013274, ISSN: 0007-1188, DOI: DOI:10.1038/SJ.BJP.0702995
- MAGYAR KALMAN ET AL: "The fate of (-)-1-(benzofuran-2-yl)-2-propylaminopenta necntdotHCl, (-)-BPAP, in rats, a potent enhancer of the impulse-evoked release of catecholamines and serotonin in the brain", EUROPEAN JOURNAL OF DRUG METABOLISM AND PHARMACOKINETICS, vol. 27, no. 3, 2002, pages 157-161, XP009144761, ISSN: 0378-7966

## Description

### TECHNICAL FIELD

The present invention relates to a composition comprising an effective dose of a racemate or an optically active substance of 1-(benzofuran-2-yl)-2-propylaminopentane represented by the following formula (1) or pharmaceutically acceptable salt thereof and a vehicle for use in the treatment of Alzheimer disease, Parkinson's disease, depression, psychosis or drug dependence, wherein the composition is to be administered transdermally.

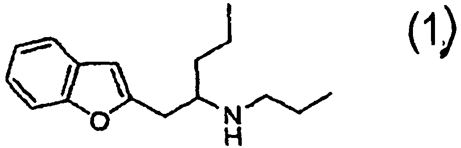

### BACKGROUND ART

A racemate or an optically active substance of the present compound 1-(benzofuran-2-yl)-2-propylaminopentane or a pharmacologically acceptable salt thereof is excellent in CAE/SAE (Catecholaminergic and Serotoninergic Activity Enhancer) effect that is a catecholaminergic activity-enhancing effect by a voltage-dependent exocytosis, different from catecholamine substitution-type release-promoting action, and is thus characterized **in that** it is hard to induce amine depletion at catecholamine nerve terminals and the release of excessive catecholamine observed in conventional monoamine oxidase inhibitors, catecholamine uptake inhibitors, and psychostimulants such as amphetamine (see Non-Patent Documents 1 and 2). Accordingly, the present compound exhibits an excellent effect as a safe and useful antidepressant, psychoactive drug, antiparkinson drug or anti-Alzheimer's drug, with fewer problems such as adverse effects including abnormal increase in behavior activity (excitatory action), neurotoxicity in the central nerve, and impaired responsiveness in patients (Patent Document 1). It is revealed that an R-configuration of its isomer has a particularly excellent pharmacological activity as compared with that of an S-configuration of its (+) isomer or its racemate (Patent Document 2), is promising as a therapeutic agent for drug dependence (Patent Document 3), and has an antiapoptotic action excellent in cellular protection and cell-death suppression (Patent Document 4). Further, percutaneous absorption preparations of 4-(2-methyl-1-imidazolyl)-2,2-diphenylbutylamide as the active ingredient are known from Patent Document 5.

Patent Document 1: WO1999/07667 (EP-A-0957080)
Patent Document 2: WO2000/26204
Patent Document 3: WO2006/057211
Patent Document 4: JP-A 2003-89643
Patent Document 5: WO 2006/082888 (EP-A-1844773)
Non-Patent Document 1: J. Knoll et al., Brit. J. Pharm. 128(8):1723-32 (1999)
Non-Patent Document 2: K. Magyar et al.; Eur. J. of Drug Metabolisms and Pharmacokinetics 27(3):157-161 (2002)

### DISCLOSURE OF THE INVENTION

### PROBLEMS TO BE SOLVED BY THE INVENTION

Generally, a drug is administered mostly as an oral drug. For exhibiting its effect, however, bioavailability of its active ingredient becomes a problem. Usually, the oral drug is administered for the purpose of absorption of its active ingredient at sites in digestive organs from a stomach to a small intestine or a colon. In the case of the oral drug, therefore, its active ingredient after absorption via digestive organs such as the stomach, the small intestine and the colon will, prior to systemic circulation, pass in initial circulation through a liver where the active ingredient undergoes liver metabolism called a first pass effect by which a reduction in its bioavailability results.

Due to a reduction in an effective dose by the first pass effect, the oral drug should sometimes be administered in a dose that is several times to several ten times as large as its originally necessary dose.
Administration of the drug in such a large dose may lead to an increase in its metabolites as originally undesired byproducts or to an abnormal increase in the blood level of the active ingredient in rare patients with deficiency of hepatic metabolizing enzymes. Such an increase in byproducts and abnormal increase in the blood level of the active ingredient in the drug may cause development of unexpected adverse effects.

On the other hand, an injection is excellent as an administration agent which unlike the oral drug, can circumvent the first pass effect in the liver and enhance bioavailability. However, the injection is an agent which causes the blood level of its active ingredient to be easily rapidly changed upon administration and makes invasion of the body inevitable upon administration. Therefore, when the injection is not suitably given, it may cause infections and damages, thus laying pains or physical burdens on patients. Moreover, treatment for depression, psychosis, Parkinson's disease, Alzheimer disease or drug dependence often extends over a long time, but when an invasive parenteral agent such as an injection is to be administered, the patient must frequently visit a hospital every time he undergoes administration because administration by a doctor or a nurse is usually necessary, and therefore, the patient is subjected to restriction of daily activity over a long period of time and has a major problem in quality of life (Q01.) of the patient.

### MEANS FOR SOLVING THE PROBLEM

The present inventors made an extensive study. As a result, the inventors found that the present compound has an unexpectedly excellent mucosal and cutaneous permeability without invasion of skin and mucous membrane and shows high transferability to the brain that is its original target site. Based on this finding, the inventors made further study and completed the present invention that has solved the problems of the injection and oral drug described above.

In other words, the present Invention relates to a composition comprising an effective dose of a racemate or an optically active substance of 1-(benzofuran-2-yl)-2-propylaminopentane represented by the following formula (1) or pharmaceutically acceptable salt thereof and a vehicle for use in the treatment of Alzheimer disease, Parkinson's disease, depression, psychosis or drug dependence, wherein the composition is to be administered transdermally.

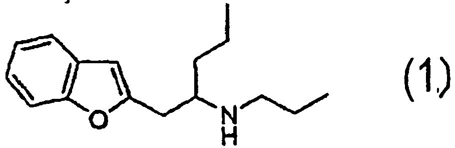

The present compound 1-(benzofuran-2-yl)-2-propylaminopentane occurs as an R-configuration of the optically active substance, that is, (-)-1-(benzofuran-2-yl)-2-propylaminopentane and an S-configuration of the optically active substance, that is,
(+)-1-(benzofuran-2-yl)-2-propylaminopentane. The compound of the present invention may be a mixture of its pure optically active substances or racemates or another mixture, but is particularly preferably the optically active substance (-)-1-(benzofuran-2-yl)-2-propylaminopentane.

Processes for producing the present compound are known. For example, W01999/07667 discloses a process for producing its racemates, and W02000/26204 and WO2001/07704 disclose a method of obtaining its optically active substances.

The pharmacologically acceptable salt of the present compound includes, for example, salts with inorganic acids such as hydrochloric acid, sulfuric acid, hydrobromic acid, nitric acid and methanesulfonic acid, and salts with organic acids such as gluconic acid, tartaric acid, maleic acid, fumaric acid, succinic acid, malic acid, citric acid and mandelic acid, among which hydrochloride is particularly preferable.

The present compound may be a free base or the pharmacologically acceptable salt, but is preferably kept in the state of a free base in a preparation, because the free base is usually superior to the salt in skin and mucous-membrane permeability.

To keep the present compound in the state of a free base in a preparation, the free base may be used as an active pharmaceutical ingredient in a preparation, or the salt may be used as an active pharmaceutical ingredient and, before or after formulation, neutralized by mixing under stirring with a basic compound generally used as a pH adjusting agent, for example, an organic base such as diethanolamine, triethanolamine, diisopropanolamine or methylethanolamine, or an inorganic base such as potassium hydroxide, sodium hydroxide, calcium hydroxide, magnesium hydroxide, trisodium phosphate, or tripotassium phosphate, thereby forming a free base of the present compound.

The administration site of the composition for transdermal or transmucosal administration in the present invention is the skin or mucous membrane, and unlike intravenous, subcutaneous, intramuscular or intraperitoneal injections or drip infusions or incision administration, the composition of the present invention upon administration does not injure the patient's body. In other words, the composition of the present invention is intended to be absorbed via sites other than digestive organs, and can be used to administer the present compound by permeation via the administration site that is the skin or the mucous membrane of the nasal cavity, oral cavity, esophagus, rectum, vagina, lung or bronchus.

The composition for transdermal or transmucosal administration in the present invention may be in the form of a liquid, semisolids or solids and may be combined if necessary with generally used additives such as an excipient, a binder, a lubricant, a disintegrator, a humectant, a diluent, a solubilizer, an isotonic agent, a suspending agent, an emulsifier, a stabilizer, a preservative, an absorption promoter, a sweetening agent, a coloring agent and if necessary with a pH adjusting agent, and these dosage forms can be prepared by a production method using known techniques.

For example, when a composition for transdermal administration is formed, its form is not particularly limited as long as it can be applied to the skin. Examples include adhesive preparations such as a gel, a cream, an ointment, a lotion, a cataplasm, a plaster, and a tape.
The vehicle used in the present invention, that is, a base for administration of the active ingredient used in the present invention to the body via the skin or mucous membrane, may be any material as long as it is generally used in compositions for skin or mucous membrane administration. Examples of the vehicle include oily vehicles such as white petrolatum, purified lanolin, squalane, silicon, liquid paraffin, vegetable oil, and wax, as well as aqueous vehicles such as water, glycol, and macrogol. The vehicle used in the tape includes generally used vehicles, for example an acrylic pressure sensitive adhesive, a rubber pressure sensitive adhesive, a silicon pressure sensitive adhesive, a vinyl ester pressure sensitive adhesive, and the like. The composition of the invention may further be compounded with a surfactant, a stabilizer, a preservative and the like and can be produced as an ointment, a cream, a gel or a tape by generally used methods.

For example, when a composition for intraoral administration is formed, its form is not particularly limited as long as it can be applied to the oral cavity, and the composition of the invention can be compounded with suitable additives such as an excipient, a binder, a disintegrator or the like, to form sublingual tablets, an intraoral disintegrator, buccal tablets, a powder, a syrup, a lemonade, an oleaginous, emulsion-type, or water-soluble ointment, a jelly, a liquid medicine, an elixir, a suspension, an emulsion, or a lozenge.

When an intraoral disintegrator is formed, it can be formed into a rapidly diffusing matrix system described in US Patent No. 5120549, a rapid diffusing administration agent consisting of a water-soluble gel capable of hydration or a foam porous skeleton structure described in US Patent No. 5079018, or a solid rapidly diffusing administration agent consisting of a water-soluble or water-dispersible carrier network structure described in UK Patent No. 1548022.

When a suppository is formed, the vehicle used in the suppository may be any material as long as it is generally used in suppositories, and examples of such vehicles include oily vehicles and aqueous vehicles. The oily vehicles include medium-chain fatty acid ester triglycerides, glycerin fatty acid esters, cacao butter, laurin fat, beef tallow, and hard fat, and the aqueous vehicles include macrogol, polypropylene glycol, and glycerin.

When long-term continuous and stable administration among various administration forms is intended, transdermally administered agents such as adhesive preparation are particularly preferably those administered via the skin because the long-lasting effect is increased due to the sustained release of the preparation, a rapid change in blood concentration is decreased, and an effective dose can be easily administered continuously and stably for a long period of time.
Desirably, the transdermal absorption preparation is for example in the form of a preparation excellent in handleability or in adherence or adhesion to the skin or of an adhesive preparation having a pressure sensitive adhesive layer formed on one side of a support, but may also be a preparation having an adherence-free surface to be contacted with the skin. The preparation in this case may be fixed for example with a tape via which it can be kept in contact with the skin.

The pressure sensitive adhesive is preferably a (meth)acrylic pressure sensitive adhesive, a rubber pressure sensitive adhesive or a silicon pressure sensitive adhesive, which has adhesiveness at ordinary temperatures and exhibits less dermal irritation upon contact with the skin.

The composition for transdermal and transmucosal administration in the present invention, which is capable of administration without invasion of skin and mucous membrane, can be used safely and conveniently as a prophylactic or therapeutic agent for central nervous system diseases, such as an anti-Alzheimer's drug, an antiparkinson drug, an antidepressant, a psychoactive drug, or a therapeutic agent for drug dependence.

The effective dose of the compound used in the present invention varies depending on the symptoms, weight and age of the patient, the administration method and the dosage form. Usually, the compound can be administered to an adult in an amount of about 0.1 to 500 mg once a day, in divided portions per day, or once every few days.

### EFFECTS OF THE INVENTION

The active ingredient administered via the skin or mucous membrane by using the composition for transdermal or transmucosal administration in the present invention does not pass through the liver in initial circulation and is thus free of the disadvantage of the reduction in an effective dose caused by the first pass effect, so that a sufficient effective dose can stably reach the body or the brain. Accordingly, even those patients who have difficulty in oral administration can, without being subjected to the influence of a meal ingested, be administered with the composition of the present invention, without increasing its metabolites as originally undesired byproducts or without abnormally increasing the blood level of the active ingredient in patients with deficiency of hepatic metabolizing enzymes. Moreover, the composition of the present invention does not invade the patient's body, thus causing no risk of pains, damages or infections, can easily regulate the administration period, frequency, or administration dose, depending on necessity for either short-term administration or long-term continuous administration, and makes hospital visits unnecessary because the patient can administer it safely by himself.

### BEST MODE FOR CARRYING OUT THE INVENTION

Hereinafter, the present invention will be described in more detail with reference to the Examples

### Examples

### Preparation Examples 1 to 6

A carboxyvinyl polymer, that is, Highvis Wako 105 (trademark) or Highvis Wako 103 (trademark), and triethanolamine were dissolved in water, and the (-) isomer of the present compound (hydrochloride) was dissolved in ethanol. Both solutions were mixed uniformly to yield gel preparations having the formulations shown in Table 1 (hereinafter, numerical values in each table are expressed in % by weight).

**Table 1**

| Formulation | Preparation Example 1 | Preparation Example 2 | Preparation Example 3 | Preparation Example 4 | Preparation Example 5 | Preparation Example 6 |
|---|---|---|---|---|---|---|
| Highvis Wako 105 | 1.50 | 1.50 | 1.50 | 1.50 | 0.60 | 0.60 |
| Highvis Wako 103 | - | - | - | - | 0.90 | 0.90 |
| Triethanolamine | 2.00 | 2.00 | 2.00 | 2.00 | 2.00 | 2.00 |
| Ethanol | 40.00 | 40.00 | 40.00 | 40.00 | 40.00 | 40.00 |
| Water | 56.25 | 56.00 | 55.50 | 54.50 | 56.30 | 55.50 |
| The present compound | 0.25 | 0.50 | 1.00 | 2.00 | 0.20 | 1.00 |

### Preparation Examples 7 to 12

The (-) isomer of the present compound (free base) was added to, and uniformly mixed with, white petrolatum to yield ointments having the formulations shown in Table 2.

**Table 2**

| Formulation | Preparation Example 7 | Preparation Example 8 | Preparation Example 9 | Preparation Example 10 | Preparation Example 11 | Preparation Example 12 |
|---|---|---|---|---|---|---|
| White petrolatum | 99.78 | 99.56 | 99.13 | 98.26 | 91.27 | 82.60 |
| The present compound | 0.22 | 0.44 | 0.87 | 1.74 | 8.73 | 17.40 |

### Preparation Example 13

The (-) isomer of the present compound (hydrochloride), methyl p-oxybenzoate and propyl p-oxybenzoate were dissolved in water under heating, and propylene glycol was added to, and well mixed with, the resulting solution. The mixture was added to an aqueous solution of Highvis Wako 105 (trademark) and triethanolamine. On the other hand, white petrolatum, stearyl alcohol, polyoxyethylene hardened castor oil 60, and glyceryl monostearate were dissolved on a thermostat bath at 75°C. Both the solutions were mixed and well mixed under gradual cooling, to yield an ointment having the formulation shown in Table 3.

**Table 3**

| Formulation | Preparation Example 13 |
|---|---|
| White petrolatum | 12.50 |
| Stearyl alcohol | 10.00 |
| Propylene glycol | 12.50 |
| Polyoxyethylene hardened castor oil 60 | 2.00 |
| Glyceryl monostearate | 0.50 |
| Methyl p-oxybenzoate | 0.10 |
| Propyl p-oxybenzoate | 0.10 |
| Highvis Wako 105 | 1.00 |
| Triethanolamine | 1.50 |
| Water | 58.80 |
| The present compound | 1.00 |

### Preparation Examples 14 to 16

Methyl p-oxybenzoate and propyl p-oxybenzoate were dissolved in water under heating, and propylene glycol was added to, and well mixed with, the solution. On the other hand, the (-) isomer of the present compound (free base), white petrolatum, stearyl alcohol, polyoxyethylene hardened castor oil 60, and glyceryl monostearate were dissolved on a thermostat bath at 75°C. Both solutions were mixed and well mixed under gradual cooling, to yield ointments having the formulations shown in Table 4.

**Table 4**

| Formulation | Preparation Example 14 | Preparation Example 15 | Preparation Example 16 |
|---|---|---|---|
| White petrolatum | 25.00 | 25.00 | 25.00 |
| Stearyl alcohol | 20.00 | 20.00 | 20.00 |
| Propylene glycol | 12.00 | 12.00 | 12.00 |
| Polyoxyethylene hardened castor oil 60 | 4.00 | 4.00 | 4.00 |
| Glyceryl monostearate | 1.00 | 1.00 | 1.00 |
| Methyl p-oxybenzoate | 0.10 | 0.10 | 0.10 |
| Propyl p-oxybenzoate | 0.10 | 0.10 | 0.10 |
| Water | 37.36 | 36.92 | 36.05 |
| The present compound | 0.44 | 0.88 | 1.75 |

### Preparation Example 17

Glycerin, propylene glycol, isopropyl myristate, the (-) isomer of the present compound (free base), and Highvis Wako 105 (trademark) were mixed uniformly with one another. Kaolin was added to, and uniformly mixed with, the mixture and then spread on a sheet (nonwoven fabric) to yield adhesive preparations having the formulations shown in Table 5.

### Preparation Example 18

Glycerin, propylene glycol, and the (-) isomer of the present compound (free base) were weighed, and Highvis Wako 105 (trademark) was added to and mixed uniformly with them. Kaolin and Alonvis AH-105 (trademark), that is, a partially neutralized product of polyacrylic acid, were added to and mixed with the mixture, and then water was added to and uniformly mixed with the mixture which was then spread on a sheet (nonwoven fabric) to yield adhesive preparations having the formulations shown in Table 5.

**Table 5**

| Formulation | Preparation Example 17 | Preparation Example 18 |
|---|---|---|
| Glycerin | 24.35 | 20.45 |
| Propylene glycol | 11.69 | 14.61 |
| Isopropyl myristate | 3.90 | - |
| Highvis Wako 105 | 3.90 | 3.90 |
| Alonvis AH-105 | - | 9.74 |
| Kaolin | 53.57 | 38.96 |
| Water | - | 9.74 |
| The present compound | 2.60 | 2.60 |

### Preparation Example 19

0.0719 g of hydroxypropyl cellulose was dissolved in 1.5 mL water. To this solution were added 0.25 g of the (-) isomer of the present compound (hydrochloride), 0.0095 g of citric acid monohydrate, 0.1241 g of β-cyclodextrin, and 0.1905 g of trehalose, and the mixture was sufficiently mixed. A solution of 0.0095 g sodium chloride in 0.05 mL water was added to the mixture. A mixture consisting of 1.4322 g of D-mannitol, 1.6566 g of lactose, 1.242 g of trehalose, and 0.0095 g of light silicic anhydride was mixed with the mixture and dried at about 50°C. After drying, the sample was pulverized, admixed, then passed twice through No. 30 sieve, and 0.0048 g of light silicic anhydride was added to and sufficiently mixed with the product. The mixture was passed 3 times through No. 30 sieve, to yield a powder preparation for intraoral administration.

### Preparation Examples 20 to 25

Each base was melted under heating on a water bath at 70°C. The (-) isomer of the present compound (hydrochloride) was added to and mixed with the melted base and then molded to give suppositories having the formulations shown in Table 6.

**Table 6**

| Formulation | Preparation Example 20 | Preparation Example 21 | Preparation Example 22 | Preparation Example 23 | Preparation Example 24 | Preparation Example 25 |
|---|---|---|---|---|---|---|
| Macrogol 400 | 27.75 | 29.78 | 29.10 | 46.25 | 49.63 | 48.50 |
| Macrogol 4000 | 37.00 | 39.70 | 38.80 | - | - | - |
| Macrogol 6000 | 27.75 | 29.78 | 29.10 | 46.25 | 49.63 | 48.50 |
| The present compound | 7.50 | 0.75 | 3.00 | 7.50 | 0.75 | 3.00 |

### Preparation Examples 26 to 28

Hard fat, Pharmasol B-115 (trademark), was melted by heating on a water bath at 37°C. The (-) isomer of the present compound (free base) was added to and mixed with the melted hard fat and then molded at about 4°C to give suppositories having the formulations shown in Table 7.

**Table 7**

| Formulation | Preparation Example 26 | Preparation Example 27 | Preparation Example 28 |
|---|---|---|---|
| Hard fat | 93.47 | 99.35 | 97.40 |
| The present compound | 6.53 | 0.65 | 2.60 |

### Preparation Example 29

Glycerin, propylene glycol, isopropyl myristate, Highvis Wako 105 (trademark) and the racemate of the present compound (hydrochloride) were mixed uniformly with one another. Kaolin was added to and mixed with the mixture and then spread on a sheet (nonwoven fabric) to yield an adhesive preparation having the formulation shown in Table 8.

**Table 8**

| Formulation | Preparation Example 29 |
|---|---|
| Glycerin | 24.25 |
| Propylene glycol | 11.64 |
| Isopropyl myristate | 3.88 |
| Highvis Wako 105 | 3.88 |
| Kaolin | 53.35 |
| The present compound | 3.00 |

### Preparation Example 30

Glycerin, propylene glycol, and the racemate of the present compound (hydrochloride) were weighed, and Highvis Wako 105 (trademark) was added to and mixed uniformly with them. Kaolin and Alonvis AH-105 (trademark) were added to and mixed with the mixture, and then water was added to and uniformly mixed with the mixture which was then spread on a sheet (nonwoven fabric) to yield an adhesive preparation having the formulation shown in Table 9.

**Table 9**

| Formulation | Preparation Example 30 |
|---|---|
| Glycerin | 20.37 |
| Propylene glycol | 14.55 |
| Highvis Wako 105 | 3.88 |
| Alonvis AH-105 | 9.70 |
| Kaolin | 38.80 |
| Water | 9.70 |
| The present compound | 3.00 |

### Preparation Example 31

0.0144 g of hydroxypropyl cellulose was dissolved in 0.5 mL water. To this solution were added 0.05 g of the racemate of the present compound (hydrochloride), 0.0019 g of citric acid monohydrate, 0.0248 g of β-cyclodextrin, and 0.0381 g of trehalose, and the mixture was sufficiently mixed. A solution of 0.0019 g sodium chloride in 0.05 mL water was added to the mixture. A mixture consisting of 0.2864 g of D-mannitol, 0.3313 g of lactose, 0.2484 g of trehalose, and 0.0019 g of light silicic anhydride was mixed with the mixture and dried at about 50°C. After drying, the sample was pulverized, admixed, then passed twice through No. 30 sieve, and 0.001 g of light silicic anhydride was added to and sufficiently mixed with the product. The mixture was further passed 3 times through No. 30 sieve, to yield a powder preparation for intraoral administration.

### Preparation Examples 32 to 33

Each base was melted by heating on a water bath at 70°C. The racemate of the present compound (hydrochloride) was added to and mixed uniformly with the melted base and then molded into suppositories having the formulations shown in Table 10.

**Table 10**

| Formulation | Preparation Example 32 | Preparation Example 33 |
|---|---|---|
| Macrogol 400 | 27.75 | 29.78 |
| Macrogol 4000 | 37.00 | 39.70 |
| Macrogol 6000 | 27.75 | 29.78 |
| The present compound | 7.50 | 0.75 |

### Preparation Examples 34 to 35

Each base was melted by heating on a water bath at 70°C. The racemate of the present compound (hydrochloride) was added to and mixed uniformly with the melted base and then molded into suppositories having the formulations shown in Table 11.

**Table 11**

| Formulation | Preparation Example 34 | Preparation Example 35 |
|---|---|---|
| Macrogol 400 | 46.25 | 49.63 |
| Macrogol 6000 | 46.25 | 49.63 |
| The present compound | 7.50 | 0.75 |

### Preparation Examples 36 to 37

Hard fat was melted by heating on a water bath at 37°C. The racemate of the present compound (hydrochloride) was added to and mixed uniformly with the melted hard fat and then molded at about 5°C into suppositories having the formulations shown in Table 12.

**Table 12**

| Formulation | Preparation Example 36 | Preparation Example 37 |
|---|---|---|
| Pharmasol B-115 | 92.5 | 99.25 |
| The present compound | 7.5 | 0.75 |

### Test Example 1

### 1) Intravenous administration

0.5 mg/kg of the (-) isomer of the present compound (hydrochloride) (1 mL/kg of 0.5 mg/mL aqueous solution in physiological saline) was administered to the tail vein of each of male SD rats fasted since the previous day, and 5 minutes, 10 minutes, 20 minutes, 30 minutes, 45 minutes, 1 hour, 1.5 hours, 2 hours, 3 hours, 4 hours, and 6 hours after administration, whole blood was collected from the abdominal aorta.

### 2) Subcutaneous administration

1 mg/kg of the (-) isomer of the present compound (hydrochloride) (5 mL/kg of 0.2 mg/mL aqueous solution) was administered subcutaneously to the back of the neck of each of male SD rats fasted since the previous day, and 5 minutes, 10 minutes, 20 minutes, 30 minutes, 1 hour, 1.5 hours, 2 hours, 3 hours, 4 hours, 6 hours, 8 hours and 10 hours after administration, whole blood was collected from the abdominal aorta.

### 3) Oral administration

10 mg/kg of the (-) isomer of the present compound (hydrochloride) (10 mL/kg of 1 mg/mL aqueous solution) was administered forcedly orally to male SD rats fasted since the previous day, and 5 minutes, 10 minutes, 20 minutes, 30 minutes, 1 hour, 1.5 hours, 2 hours, 3 hours, 4 hours, 6 hours, 8 hours and 10 hours after administration, whole blood was collected from the abdominal aorta.

### 4) Measurement of blood level

The blood collected from each rat was transferred to a blood collecting tube containing 2Na EDTA, then sufficiently mixed by turning the tube upside down, and then centrifuged at 3,000 rpm for 10 minutes, and the obtained plasma was recovered. The plasma was freeze-preserved at -20°C until analysis. The plasma level of the compound was measured by high performance liquid chromatography (HPLC). As a pharmacokinetic parameter, AUCO-t (area under the blood level-time curve, hereinafter referred to as "AUC") was calculated by a trapezoidal method, and Cmax (maximum blood concentration) and tmax (time-to-maximum blood concentration) were determined from actual measurement values.

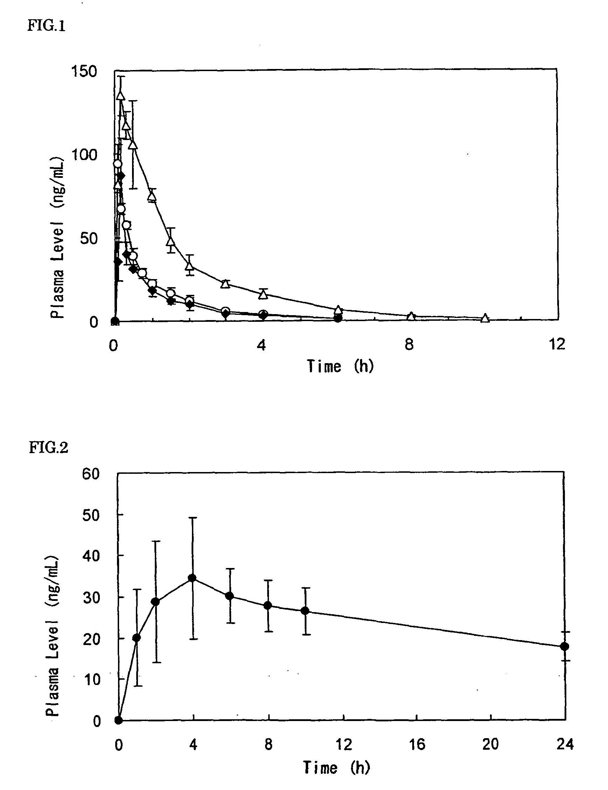

The results are shown in Table 13 and FIG. 1. The blood level in the oral administration was low as compared with the given dose, and the bioavailability F (%) was also as extremely low as about 1/24 relative to that in the intravenous administration or about 1/35 relative to that in the subcutaneous administration.

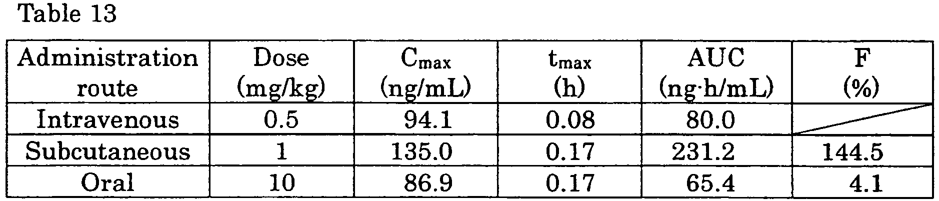

### Test Example 2

Preparation Example 15 was weighed so as to be in a dose of 10 mg/kg and then spread thin on a water-impervious sheet. This preparation was attached firmly to the dehaired abdomen of each of male SD rats anesthetized under ether. For fixing the preparation, a cut absorbent cotton was placed on the preparation and then wrapped in an adhesive bandage. After attachment, each rat was returned to a cage, and until the test was finished, the preparation was left attached to each rat. Partial blood was collected through the jugular vein with time from the individual rats 1, 2, 4, 6, 8, 10 and 24 hours after administration.

The blood collected from each rat was treated in the same manner as in Test Example 1 and measured for plasma level by high performance liquid chromatography/tandem mass spectrometry (LC/MS/MS).

The results are shown in Table 14 and FIG. 2. In the transdermal administration, the present compound showed a moderate increase in blood level and subsequent disappearance, where a rapid change in blood level such as in the intravenous administration, subcutaneous administration and oral administration in Test Example 1 was not recognized, and stable blood levels were persistently obtained. The bioavailability F (%) in intravenous administration was about 8-fold higher in the transdermal administration than in the oral administration. After administration, no abnormality in the skin was observed.

**Table 14**

| Preparation | Dose (mg/kg) | Cₘₐₓ (ng/mL) | tₘₐₓ (h) | AUC (ng·h/mL) | F (%) |
|---|---|---|---|---|---|
| Preparation Example 15 | 11.31 | 36.3 | 4.67 | 582.2 | 32.17 |

### Test Example 3

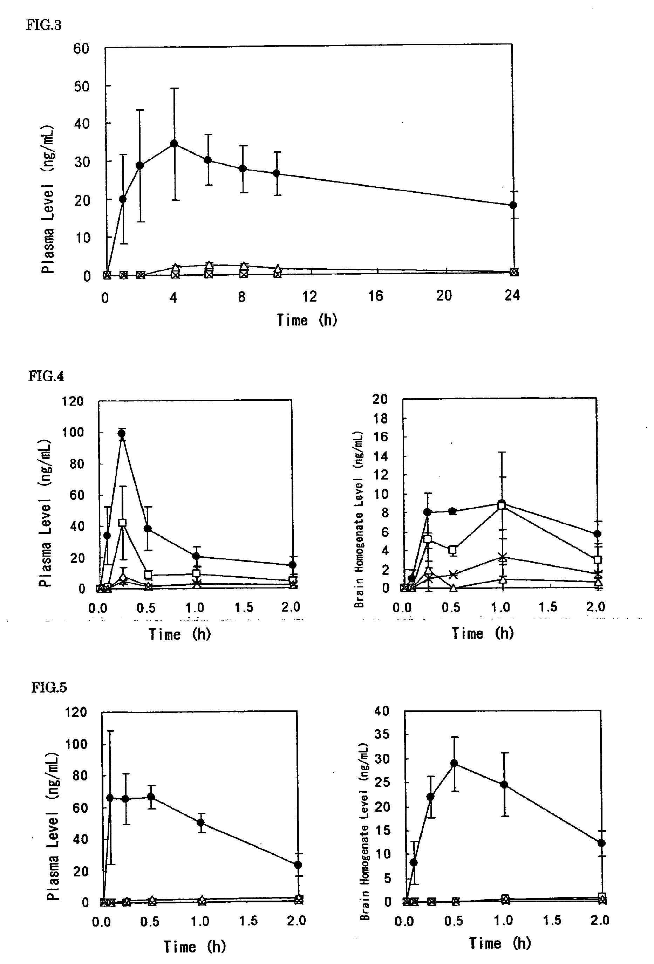

Changes in plasma level of metabolites measured simultaneously with the present compound in Test Example 2 were examined in Test Example 3, and the results of the changes in plasma level thereof, together with the unchanged drug, are shown in FIG. 3.

The concentrations of the metabolites in plasma in the transdermal administration were extremely low.

### Test Example 4

### 1) Oral administration

The (-) isomer of the present compound (hydrochloride) was administered forcedly orally in a dose of 10 mg/kg (10 mL/kg of 1 mg/mL aqueous solution) to fasted male SD rats, and 5, 15, 30, 60 and 120 minutes after administration, whole blood was collected from the abdominal aorta.

### 2) Intraoral administration (aqueous solution, powder preparation)

The (-) isomer of the present compound (hydrochloride) was dropped in a dose of 1 mg/kg (0.1 mL/kg of 10 mg/mL aqueous solution) into the oral cavity of each fasted male SD rat anesthetized under ether. In the case of the powder preparation, the preparation which had been weighed so as to be in a dose of 1 mg/kg was placed beneath the tongue of each rat under anesthesia. In either case, the rats were left for 1 minute under anesthesia and then returned to cages, and 5, 15, 30, 60 and 120 minutes after administration, whole blood was collected from the abdominal aorta.

### 3) Collection of brain samples and measurement of concentrations in plasma and brain homogenates

The blood collected from each rat was treated in the same manner as in Test Example 1. After blood collection, blood in the body of each rat was perfused with refrigerated physiological saline, and the cerebrum was excised therefrom. The cerebrum was cut thin with a knife and homogenized in 5 mL physiological saline added per gram of the tissues. 0.5 mL acetonitrile was added to 0.5 mL of the brain homogenate which was then stirred by means of a test tube mixer and centrifuged at 10,000 rpm for 5 minutes, to give a supernatant.

The concentrations of the compound in the plasma and in the brain homogenate were measured by LC/MS/MS. Simultaneously, the metabolites were quantitatively determined by using metabolite standards.

Changes in the concentration of the present compound in the plasma and changes in the concentration thereof in the average brain homogenate, in the oral administration, are shown in FIG. 4, those in the intraoral administration (aqueous solution) are shown in FIG. 5, and those in the intraoral administration (powder preparation) are shown in FIG. 6. The intraoral administration, although the dose was about 1/10 relative to that in the oral administration, showed not only a blood concentration equal to or higher than that in the oral administration, but also lower metabolites. Comparison in the concentration in the brain homogenate indicated that the transferability of the compound to the brain in the intraoral administration is significantly higher than in the oral administration.

### Test Example 5

Preparation Example 24 weighed so as to be in a dose of 1 mg/kg was administered rectally to each of male SD rats anesthetized under ether. The intestinal tract before and after the preparation was ligated such that the preparation did not move, then the opened abdominal part was sutured, and each rat was returned to a cage. Partial blood was collected through the jugular vein and collected with time from the individual rats 10 minutes, 30 minutes, 1 hour, 2 hours, 3 hours, 4 hours, 5 hours, 6 hours and 8 hours after administration. The blood collected from each rat was treated in the same manner as in Test Example 1 and measured for its changes in the concentrations of the unchanged drug and metabolites in plasma by LC/MS/MS.

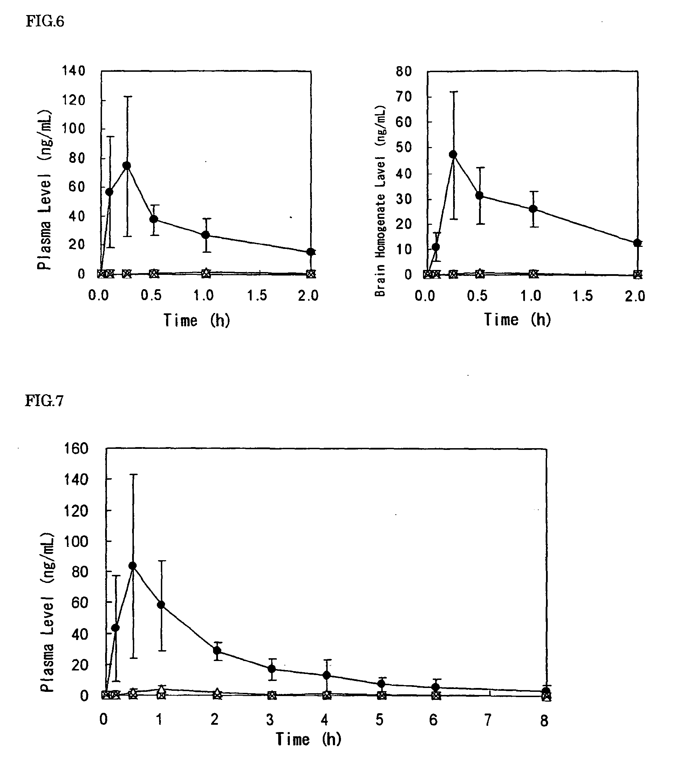

Changes in the concentrations of the present compound and its metabolites in plasma are shown in FIG. 7. Higher blood levels and lower metabolites were shown in the intrarectal administration than in the oral administration.

### Test Example 6

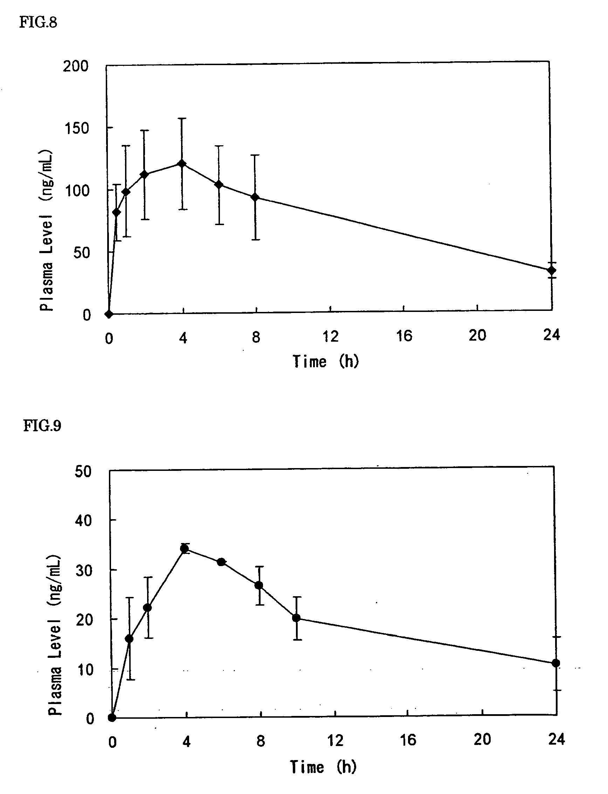

Preparation Example 13 was weighed so as to be in a dose of 10 mg/kg and then spread thin on a water-impervious sheet. Each male SD rat whose hair had been removed on the previous day was anesthetized under ether, and the sheet was attached firmly to the abdomen of the rat. Thereafter, the plasma level was measured in the same manner as in Test Example 2 except that the time point of blood collection was 0.5, 1, 2, 4, 6, 8 and 24 hours respectively after administration. A change in the plasma level of the present compound is shown in FIG. 8.

### Test Example 7

Preparation Example 9 was weighed so as to be in a dose of 10 mg/kg and then spread thin on a water-impervious sheet. Each male SD rat whose hair had been removed on the previous day was anesthetized under ether, and the sheet was attached firmly to the abdomen of the rat. Thereafter, the plasma level was measured in the same manner as in Test Example 2 except that the time point of blood collection was 1, 2, 4, 6, 8, 10 and 24 hours respectively after administration. A change in the plasma level of the present compound is shown in FIG. 9.

### Test Example 8

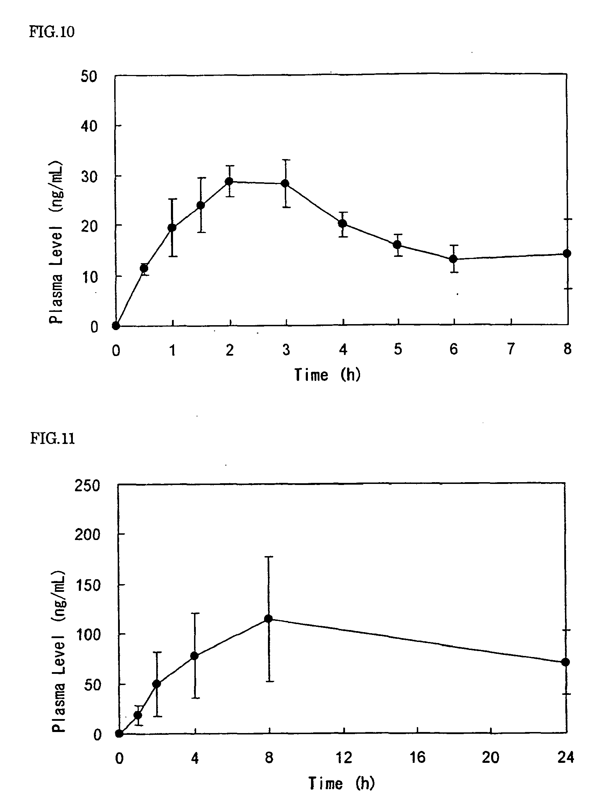

Preparation Example 5 was weighed so as to be in a dose of 1 mg/kg and then spread thin on a water-impervious sheet. Each male SD rat whose hair had been removed on the previous day was anesthetized under ether, and the sheet was attached firmly to the abdomen of the rat. Thereafter, the plasma level was measured in the same manner as in Test Example 2 except that the time point of blood collection was 0.5, 1, 1.5, 2, 3, 4, 5, 6 and 8 hours respectively after administration. A change in the plasma level of the present compound is shown in FIG. 10.

### Test Example 9

Each male SD rat whose hair had been removed on the previous day was anesthetized under ether, and a sheet on which Preparation Example 17 had been spread so as to be in a dose of 10 mg/kg was attached firmly to the abdomen of the rat. Thereafter, the plasma level was measured in the same manner as in Test Example 2 except that the time point of blood collection was 1, 2, 4, 8 and 24 hours respectively after administration.

A change in the plasma level of the present compound is shown in FIG. 11.

### INDUSTRIAL APPLICABILITY

A composition for transdermal administration according to the present invention, which includes an effective dose of a racemate or an optically acceptable substance of 1-(benzofuran-2-yl)-2-propylaminopentane or a pharmacologically acceptable salt thereof and a vehicle, can pass for example as an adhesive preparation such as a gel, a cream, an ointment, a lotion, a cataplasm, a plaster or a tape, through a skin or a mucous membrane thereby allowing an effective dose to stably reach a body or a brain and can be used as an anti-Alzheimer's drug, an antiparkinson drug, an antidepressant, a psychoactive drug or a therapeutic agent for drug dependence, which is an excellent drug free of disadvantages such as a reduction in an effective dose caused by a first pass effect in a liver, as well as pains, damages or infections.

### BRIEF DESCRIPTION OF THE DRAWINGS

FIG. 1 is a graph showing changes in a plasma level of the present compound when administered once intravenously, subcutaneously and orally (○: intravenous, Δ: subcutaneous, ◆: oral).
FIG. 2 is a graph showing a change in the plasma level of the present compound where Preparation Example 15 was transdermally administered.
FIG. 3 is a graph showing changes in the plasma levels of the present compound and its metabolites where Preparation Example 15 was transdermally administered (●: the present compound, □: metabolite 1, Δ: metabolite 2, ×: metabolite 3).
FIG. 4 is a graph showing changes in the plasma levels and brain homogenate levels of the present compound and its metabolites where the compound (aqueous solution) was orally administered (•: the present compound, □: metabolite 1, Δ: metabolite 2, ×: metabolite 3).
FIG. 5 is a graph showing changes in the plasma levels and brain homogenate levels of the present compound and its metabolites where the compound (aqueous solutions) was intraorally administered (•: the present compound, □: metabolite 1, Δ: metabolite 2, x: metabolite 3).
FIG. 6 is a graph showing changes in the plasma levels and brain homogenate levels of the present compound and its metabolites where the compound (powder) was intraorally administered (•: the present compound, □: metabolite 1, Δ: metabolite 2, x: metabolite 3).
FIG. 7 is a graph showing changes in the plasma levels of the present compound and its metabolites where Preparation Example 24 was rectally administered (●: the present compound, □: metabolite 1, Δ: metabolite 2, x: metabolite 3).
FIG. 8 is a graph showing a change in the plasma level of the present compound where Preparation Example 13 was transdermally administered.
FIG. 9 is a graph showing a change in the plasma level of the present compound where Preparation Example 9 was transdermally administered.
FIG. 10 is a graph showing a change in the plasma level of the present compound where Preparation Example 5 was transdermally administered.
FIG. 11 is a graph showing a change in the plasma level of the present compound where Preparation Example 17 was transdermally administered.

## Claims

1. A composition comprising an effective dose of a racemate or an optically active substance of 1-(benzofuran-2-yl)-2-propylaminopentane represented by the following formula (1) or pharmaceutically acceptable salt thereof 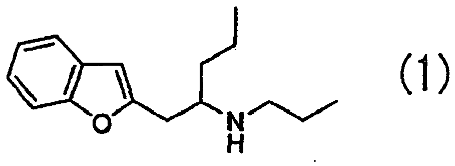 and a vehicle for use in the treatment of Alzheimer disease, Parkinson's disease, depression, psychosis or drug dependence, wherein the composition is to be administered transdermally.

2. The composition comprising an effective dose of a racemate or an optically active substance of 1-(benzofuran-2-yl)-2-propylaminopentane for use in the treatment of Alzheimer disease, Parkinson's disease, depression, psychosis or drug dependence according to claim 1, wherein the 1-(benzofuran-2-yl)-2-propylaminopentane is optically active (-)-1-(benzofuran-2-yl)-2-propylaminopentane.

3. The composition comprising an effective dose of a racemate or an optically active substance of 1-(benzofuran-2-yl)-2-propylaminopentane for use in the treatment of Alzheimer disease, Parkinson's disease, depression, psychosis or drug dependence according to claim 1 or 2, wherein the pharmacologically acceptable salt is a hydrochloride.

4. The composition comprising an effective dose of a racemate or an optically active substance of 1-(benzofuran-2-yl)-2-propylaminopentane for use in the treatment of Alzheimer disease, Parkinson's disease, depression, psychosis or drug dependence according to claim 1 or 2, wherein the 1-(benzofuran-2-yl)-2-propylaminopentane is kept in a state of a free base.

5. The composition comprising an effective dose of a racemate or an optically active substance of 1-(benzofuran-2-yl)-2-propylaminopentane for use in the treatment of Alzheimer disease, Parkinson's disease, depression, psychosis or drug dependence according to any one of claims 1 to 4, wherein the composition is in a dosage form of an adhesive preparation.

## Patentansprüche

1. Zusammensetzung, die eine wirksame Dosis eines Racemats oder einer optisch aktiven Substanz von 1-(Benzofuran-2-yl)-2-propylaminopentan, das durch die folgende Formel (1) dargestellt wird, oder ein pharmazeutisch annehmbares Salz davon 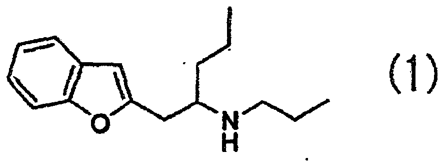 sowie einen Träger umfasst, zur Verwendung bei der Behandlung von Alzheimer-Krankheit, Parkinson-Krankheit, Depression, Psychose oder Substanzabhängigkeit, wobei die Zusammensetzung transdermal zu verabreichen ist.

2. Zusammensetzung, die eine wirksame Dosis eines Racemats oder einer optisch aktiven Substanz von 1-(Benzofuran-2-yl)-2-propylaminopentan umfasst, zur Verwendung bei der Behandlung von Alzheimer-Krankheit, Parkinson-Krankheit, Depression, Psychose oder Substanzabhängigkeit gemäß Anspruch 1, wobei das 1-(Benzofuran-2-yl)-2-propylaminopentan optisch aktives (-)-1-(Benzofuran-2-yl)-2-propylaminopentan ist.

3. Zusammensetzung, die eine wirksame Dosis eines Racemats oder einer optisch aktiven Substanz von 1-(Benzofuran-2-yl)-2-propylaminopentan umfasst, zur Verwendung bei der Behandlung von Alzheimer-Krankheit, Parkinson-Krankheit, Depression, Psychose oder Substanzabhängigkeit gemäß Anspruch 1 oder 2, wobei das pharmakologisch annehmbare Salz ein Hydrochlorid ist.

4. Zusammensetzung, die eine wirksame Dosis eines Racemats oder einer optisch aktiven Substanz von 1-(Benzofuran-2-yl)-2-propylaminopentan umfasst, zur Verwendung bei der Behandlung von Alzheimer-Krankheit, Parkinson-Krankheit, Depression, Psychose oder Substanzabhängigkeit gemäß Anspruch 1 oder 2, wobei das 1-(Benzofuran-2-yl)-2-propylaminopentan im Zustand einer freien Base gehalten wird.

5. Zusammensetzung, die eine wirksame Dosis eines Racemats oder einer optisch aktiven Substanz von 1-(Benzofuran-2-yl)-2-propylaminopentan umfasst, zur Verwendung bei der Behandlung von Alzheimer-Krankheit, Parkinson-Krankheit, Depression, Psychose oder Substanzabhängigkeit gemäß einem der Ansprüche 1 bis 4, wobei die Zusammensetzung in der Darreichungsform eines Klebepräparats vorliegt.

## Revendications

1. Composition comprenant une dose efficace d'un racémate ou d'une substance optiquement active de 1-(benzofuran-2-yl)-2-propylaminopentane représenté par la formule suivante (1) ou d'un sel pharmaceutiquement acceptable de celui-ci 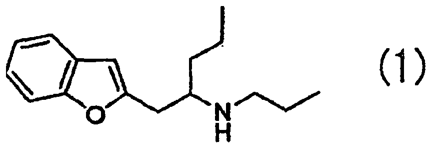 et un véhicule, pour une utilisation dans le traitement de la maladie d'Alzheimer, de la maladie de Parkinson, de la dépression, de la psychose ou de la pharmacodépendance, dans laquelle la composition est à administrer par voie transdermique.

2. Composition comprenant une dose efficace d'un racémate ou d'une substance optiquement active de 1-(benzofuran-2-yl)-2-propylaminopentane pour une utilisation dans le traitement de la maladie d'Alzheimer, de la maladie de Parkinson, de la dépression, de la psychose ou de la pharmacodépendance selon la revendication 1, dans laquelle le 1-(benzofuran-2-yl)-2-propylaminopentane est le (-)-1-(benzofuran-2-yl)-2-propylaminopentane optiquement actif.

3. Composition comprenant une dose efficace d'un racémate ou d'une substance optiquement active de 1-(benzofuran-2-yl)-2-propylaminopentane pour une utilisation dans le traitement de la maladie d'Alzheimer, de la maladie de Parkinson, de la dépression, de la psychose ou de la pharmacodépendance selon la revendication 1 ou 2, dans laquelle le sel pharmacologiquement acceptable est un chlorhydrate.

4. Composition comprenant une dose efficace d'un racémate ou d'une substance optiquement active de 1-(benzofuran-2-yl)-2-propylaminopentane pour une utilisation dans le traitement de la maladie d'Alzheimer, de la maladie de Parkinson, de la dépression, de la psychose ou de la pharmacodépendance selon la revendication 1 ou 2, dans laquelle le 1-(benzofuran-2-yl)-2-propylaminopentane est maintenu à l'état de base libre.

5. Composition comprenant une dose efficace d'un racémate ou d'une substance optiquement active de 1-(benzofuran-2-yl)-2-propylaminopentane pour une utilisation dans le traitement de la maladie d'Alzheimer, de la maladie de Parkinson, de la dépression, de la psychose ou de la pharmacodépendance selon l'une quelconque des revendications 1 à 4, dans laquelle la composition se présente sous une forme pharmaceutique de préparation adhésive.
